# EUROPEAN PATENT APPLICATION

(11) **EP 4 814 799 A1**
(43) Date of publication of application: **30.09.2026**
(21) Application number: 25199882.9
(22) Date of filing: 03.09.2025
(51) Int. Cl.: G06N 3/06, G06N 3/065, G06N 3/049, A61N 1/36

(54) **SPIKE SIGNAL GENERATION DEVICE AND SPIKE SIGNAL PROCESSING METHOD FOR BRAIN-COMPUTER INTERFACE**

(30) Priority: 28.03.2025 KR 20250040206
(71) Applicant: Korea Advanced Institute of Science and Technology, Daejeon 34141 (KR)
(72) Inventor: Choi, Yang-Kyu, 34141 Daejeon (KR); Son, Joon-Ha, 34141 Daejeon (KR)
(74) Representative: Ström & Gulliksson AB

(57) **Abstract**

A spike signal generation device according to one embodiment of the present disclosure includes a signal input unit, an artificial neuron, and a signal output unit. The signal input unit includes a signal input electrode, and an input signal conversion unit. The signal input electrode is configured to transmit an input spike signal output from a signal output neuron. The input spike signal is input from the input signal conversion unit, and an input signal different from the input spike signal is output. The artificial neuron includes an input semiconductor unit, an output semiconductor unit, and a floating semiconductor unit. The input semiconductor unit is configured to receive the input signal. In case that a voltage applied to the input semiconductor unit is greater than or equal to a reference voltage, an output spike signal is output from the output semiconductor unit. The signal output unit includes an output signal conversion unit, and a signal output electrode. The output signal conversion unit receives the output spike signal to output an output signal different from the output spike signal. The signal output electrode is configured to transmit the output signal to the signal input neuron.

## Description

### BACKGROUND

### 1. Technical Field

The present disclosure relates to a spike signal generation device and a spike signal processing method. More particularly, the present disclosure relates to a spike signal generation device and a spike signal processing method for detecting a spike signal generated from biological neurons, generating corresponding spike signal, and transmitting the spike signal to the biological neurons.

### 2. Description of the Related Art

An artificial neuron may be a neuromorphic device that mimics behaviors of a biological neuron. The biological neuron may detect stimuli. In case that the detected stimuli exceeds a threshold, the biological neuron may output a biological spike signal. An input signal may be applied to an artificial neuron. In case that the input signal exceeds a threshold, the artificial neuron may output an artificial spike signal.

However, it may be challenging for a spike signal to be directly transmitted between a biological neuron and an artificial neuron. For example, a biological spike signal of a biological neuron on a microelectrode array may typically be around a 100 µV level. However, an artificial spike signal of an artificial neuron may typically be greater than or equal to 1 V. That is, amplitudes of the biological spike signal and the artificial spike signal may differ. Accordingly, it may be challenging for an artificial neuron to receive a biological spike signal or for a biological neuron to receive an artificial spike signal.

Therefore, there is a need for a device and a system capable of transmitting a spike signal between a biological neuron and an artificial neuron.

### SUMMARY

The present disclosure is to resolve the above challenges and relates to a spike signal generation device and a spike signal processing method using a signal input unit and a signal output unit to allow real-time transmission of a spike signal between a biological neuron and an artificial neuron.

In inputting a signal of the present disclosure, a signal input unit may be used to input a biological spike signal to an artificial neuron.

In outputting a signal of the present disclosure, a signal output unit may be used to input an artificial spike signal to a biological neuron.

A spike signal generation device according to one embodiment of the present disclosure may include a signal input unit, an artificial neuron and a signal output unit. The signal input unit may include a signal input electrode and an input signal conversion unit. The signal input electrode may be configured to transmit an input spike signal output from a signal output neuron. The input signal conversion unit may receive the input spike signal to output an input signal different from the input spike signal. The input signal conversion unit may include an input operational amplifier and an input adjustment resistor. The artificial neuron may include an input semiconductor unit, an output semiconductor unit and a floating semiconductor unit. The input semiconductor unit may be configured to receive the input signal. The output semiconductor unit may be disposed to be spaced apart from the input semiconductor unit. The floating semiconductor unit may be disposed between the input semiconductor unit and the output semiconductor unit. In case that a voltage of the input semiconductor unit is equal to or greater than a reference voltage, the output semiconductor unit may output an output spike signal. The signal output unit may include an output signal conversion unit and a signal output electrode. The output signal conversion unit may be configured to receive the output spike signal to output an output signal different from the output spike signal. The signal output electrode may be configured to transmit the output signal to a signal input neuron. The output signal conversion unit may include an output adjustment resistor.

In one embodiment of the present disclosure, the input signal conversion unit may further include an input series resistor and an input parallel resistor. An input electrode of the input series resistor may be configured to receive the input spike signal. An output electrode of the input series resistor may be connected to an inverting input electrode of the input operational amplifier. An input electrode of the input parallel resistor may be connected to the inverting input electrode of the input operational amplifier. An output electrode of the input parallel resistor may be connected to a signal output electrode of the input operational amplifier. A non-inverting input electrode of the input operational amplifier may be connected to ground.

In one embodiment of the present disclosure, the output signal conversion unit may further include an output transistor. A control electrode of the output transistor may be connected to an output electrode of the output adjustment resistor.

In one embodiment of the present disclosure, the signal input unit may further include an input signal integration unit configured to transmit the input signal to the input semiconductor unit. The input signal integration unit may include an input capacitor. An input electrode of the input capacitor may be connected to the input semiconductor unit. The signal output unit may further include an output signal integration unit configured to transmit the output signal to the signal output electrode. The output integration unit may include an output capacitor and an output resistor. An input electrode of the output capacitor and an input electrode of the output resistor may be connected to an output electrode of the output transistor.

In one embodiment of the present disclosure, the input adjustment resistor and the output adjustment resistor may be variable resistors whose resistance values can be adjusted. The signal output electrode of the input operational amplifier may be connected to an input electrode of the input adjustment resistor. The output semiconductor unit may be connected to an input electrode of the output adjustment resistor.

In one embodiment of the present disclosure, the floating semiconductor unit may be doped into any one type of an n-type and a p-type. The input semiconductor unit and the output semiconductor unit may be doped into one of the n-type and the p-type, different from that of the floating semiconductor unit.

In one embodiment of the present disclosure, the artificial neuron may further include a gate insulating layer and a gate electrode. The gate insulating layer may be disposed on the floating semiconductor unit. The gate electrode may be disposed on the gate insulating layer. A voltage may be applied to the gate electrode to adjust the reference voltage.

In one embodiment of the present disclosure, the input signal conversion unit may further include a first current mirror and a second current mirror configured to copy a received current. An input electrode of the first current mirror may be connected to an output electrode of the input adjustment resistor. An input electrode of the second current mirror may be connected to a copy electrode of the first current mirror. A copy electrode of the second current mirror may be connected to the input semiconductor unit. The copy electrode of the first current mirror may be configured to copy a current of the input electrode of the first current mirror. The copy electrode of the second current mirror may be configured to copy a current of the input electrode of the second current mirror.

In one embodiment of the present disclosure, an output electrode of the input capacitor, an output electrode of the output capacitor, and an output electrode of the output resistor may be connected to ground.

In one embodiment of the present disclosure, the signal input electrode and the signal output electrode may be a micro electrode array.

In one embodiment of the present disclosure, the input signal conversion unit may be provided in a plurality. The input signal output from a first input signal conversion unit among the plurality of input signal conversion units may be a first conversion input signal. The input signal output from a second input signal conversion unit among the plurality of input signal conversion units may be a second conversion input signal. The input signal transmitted from the output signal integration unit to the input semiconductor unit may be an integration input signal formed by integration of the first conversion input signal and the second conversion input signal.

In one embodiment of the present disclosure, the output signal conversion unit may be provided in a plurality. The output signal output from a first output signal conversion unit among the plurality of output signal conversion units may be a first conversion output signal. The output signal output from a second output signal conversion unit among the plurality of output signal conversion units may be a second conversion output signal. The output signal transmitted from the output signal integration unit to the signal output electrode may be an integrated output signal formed by integration of the first conversion output signal and the second conversion output signal.

In one embodiment of the present disclosure, the input signal conversion unit, the input signal integration unit, the artificial neuron and the output signal conversion unit may be provided in a plurality. A first input signal integration unit among the plurality of input signal integration units may be configured to transmit a first conversion input signal output from a first input signal conversion unit among the plurality of input signal conversion units and a second conversion input signal output from a second input signal conversion unit among the plurality of input signal conversion units to a first artificial neuron among the plurality of artificial neurons. A second input signal integration unit among the plurality of input signal integration units may be configured to transmit a third conversion input signal output from a third input signal conversion unit among the plurality of input signal conversion units and a fourth conversion input signal output from a fourth input signal conversion unit among the plurality of input signal conversion units to a second artificial neuron among the plurality of artificial neurons. A first output spike signal output from the first artificial neuron may be input to a first output signal conversion unit among the plurality of output signal conversion units. A second output spike signal output from the second artificial neuron may be input to a second output signal conversion unit among the plurality of output signal conversion units. The output signal integration unit may be configured to transmit a first conversion output signal output from the first output signal conversion unit and a second conversion output signal output from the second output signal conversion unit to the signal output electrode.

A spike signal processing method according to one embodiment of the present disclosure may include inputting a signal, generating a signal and outputting a signal. In the inputting a signal, an input spike signal output from a signal output neuron may be detected by a signal input electrode. In the inputting a signal, the input spike signal may be input to an input signal conversion unit including an input operational amplifier and an input adjustment resistor to output a conversion input signal different from the input spike signal. In the inputting a signal, the conversion input signal may be input to an input signal integration unit including an input capacitor to transmit an integration input signal. In the generating a signal, the integration input signal may be input to an artificial neuron including an input semiconductor unit, an output semiconductor unit spaced apart from the input semiconductor unit, and a floating semiconductor unit disposed between the input semiconductor unit and the output semiconductor unit. In the generating a signal, in case that a voltage of the input semiconductor unit is greater than or equal to a reference voltage, an output spike signal may be output from the artificial neuron. In the outputting a signal, the output spike signal may be input to an output signal conversion unit including an output adjustment resistor to output a conversion output signal different from the output spike signal. In the outputting a signal, the conversion output signal may be input to an output signal integration unit including an output capacitor and an output resistor to transmit an integrated output signal. In the outputting a signal, the integrated output signal may be transmitted from a signal output electrode to a signal input neuron.

In one embodiment of the present disclosure, the generating a signal may include awaiting signal generation and generating a spike signal. In the awaiting signal generation, a voltage of the input semiconductor unit may be less than the reference voltage. In the generating a spike signal, the voltage of the input semiconductor unit may be greater than or equal to the reference voltage to output an output spike signal, and a voltage of the integration input signal may be reduced to below the reference voltage.

In one embodiment of the present disclosure, adjusting an input signal and adjusting an output signal may be further included. In the adjusting an input signal, a resistance value of the input adjustment resistor may be adjusted to control the conversion input signal. In the adjusting an output signal, a resistance value of the output adjustment resistor may be adjusted to control the conversion output signal.

In one embodiment of the present disclosure, adjusting a reference voltage may be further included. The artificial neuron may further include a gate insulating layer disposed on the floating semiconductor unit and a gate electrode disposed on the gate insulating layer. In the adjusting a reference voltage, a voltage may be applied to the gate electrode to adjust the reference voltage.

In one embodiment of the present disclosure, the signal output neuron and the signal input neuron may be biological neurons.

According to one embodiment of the present disclosure, a signal input unit and a signal output unit may be utilized for real-time transmission of a spike signal between a biological neuron and an artificial neuron.

In one embodiment of the present disclosure, in the inputting a signal, a signal input unit may be utilized to input a biological signal to an artificial neuron.

In one embodiment of the present disclosure, a signal output unit may be utilized to input an artificial spike neuron to a biological neuron.

### BRIEF DESCRIPTION OF THE DRAWINGS

These and/or other features will become apparent and more readily appreciated from the following description of the embodiments, taken in conjunction with the accompanying drawings in which:
FIG. 1 is an exemplary block diagram of a signal transmission system according to one embodiment of the present disclosure;
FIG. 2 is an exemplary block diagram of a signal generation device according to one embodiment of the present disclosure;
FIG. 3 is an exemplary block diagram of a signal input unit according to one embodiment of the present disclosure;
FIG. 4 is an exemplary block diagram of a signal output unit according to one embodiment of the present disclosure;
FIG. 5 is an exemplary circuit diagram of a signal transmission system according to one embodiment of the present disclosure;
FIG. 6A is an exemplary circuit diagram of an input inverting amplifier circuit according to one embodiment of the present disclosure;
FIG. 6B is an exemplary circuit diagram of an input signal conversion circuit according to one embodiment of the present disclosure;
FIG. 6C is an exemplary circuit diagram of an input signal integration unit according to one embodiment of the present disclosure;
FIG. 6D is an exemplary illustration of an artificial neuron according to one embodiment of the present disclosure;
FIG. 6E and FIG. 6F are exemplary circuit diagrams of an output signal conversion unit according to one embodiment of the present disclosure;
FIG. 6G is an exemplary circuit diagram of an output signal integration unit according to one embodiment of the present disclosure;
FIG. 7A is an exemplary block diagram of a signal input unit according to one embodiment of the present disclosure;
FIG. 7B is an exemplary circuit diagram of an input signal integration unit according to one embodiment of the present disclosure;
FIG. 8A is an exemplary block diagram of a signal output unit according to one embodiment of the present disclosure;
FIG. 8B is an exemplary circuit diagram of an output signal integration unit according to one embodiment of the present disclosure;
FIG. 9A is an exemplary block diagram of a portion of a signal generation device according to one embodiment of the present disclosure;
FIG. 9B is an exemplary block diagram of another portion of a signal generation device according to one embodiment of the present disclosure;
FIG. 10 is an exemplary block diagram of a signal transmission system according to one embodiment of the present disclosure;
FIG. 11 is an exemplary flow chart of a signal processing method according to one embodiment of the present disclosure;
FIG. 12 is an exemplary flow chart of a signal processing method according to one embodiment of the present disclosure;
FIG. 13A is a microscopic image of a signal output neuron and a signal input electrode according to one experimental example of the present disclosure;
FIG. 13B is an input spike signal graph according to one experimental example of the present disclosure;
FIG. 14A is a graph of a voltage of an input semiconductor unit and an output spike signal by a constant current signal according to one experimental example of the present disclosure;
FIG. 14B is a graph of an input spike signal and an output spike signal according to one experimental example of the present disclosure;
FIG. 14C is a graph of a plurality of input spike signals, and a voltage of an input semiconductor unit and an output spike signal by the plurality of input spike signals according to one experimental example of the present disclosure;
FIG. 15A is a graph of an output spike signal, an integration output signal, and a signal input neuron spike signal according to one experimental example of the present disclosure;
FIG. 15B and FIG. 15C are graphs of an integration output signal and a signal input neuron spike signal according to one experimental example of the present disclosure;
FIG. 15D is a graph of an output spike signal, an integration output signal, and a signal input neuron spike signal according to one experimental example of the present disclosure;
FIG. 15E and FIG. 15F are graphs of an integration output signal and a signal input neuron spike signal according to one experimental example of the present disclosure;
FIG. 16 is a graph of an input spike signal, a voltage of an input semiconductor unit, an output spike signal, and a signal input neuron spike signal according to one experimental example of the present disclosure;
FIG. 17 is an exemplary schematic diagram illustrating a signal transmission effects by a signal generation device of the present disclosure; and
FIG. 18 is an exemplary schematic diagram illustrating a biological neuron replacement function of a signal generation device of the present disclosure.

### DETAILED DESCRIPTION

References will now be made in detail to certain embodiments, of which examples are illustrated in the accompanying drawings, where like reference numerals refer to like elements throughout. The embodiments may have a variety of forms and permutations, but the present disclosure shall by no means be construed as being limited to the described embodiments. Rather, the present disclosure shall be construed to encompass all forms, permutations, equivalents and substitutes covered by the technical ideas and scope of the present disclosure. Accordingly, the embodiments are merely described below, by referring to the figures, to explain features of the present disclosure.

Like or identical reference numerals refer to like or identical elements. Moreover, in the accompanying drawings, the thicknesses, ratios, and dimensions of the elements may not be to exact scale and may have been exaggerated for the benefit of effective explanation of the technical features associated with these elements. As such, the present disclosure shall not be restricted to the thicknesses, ratios, dimensions, etc. illustrated in the drawings.

An expression such as "comprising" or "including" is intended to designate a characteristic, a number, a step, an operation, an element, a part or combinations thereof, and shall not be construed to preclude any possibility of presence or addition of one or more other characteristics, numbers, steps, operations, elements, parts or combinations thereof.

A description such as "a voltage of a first element" includes all a voltage applied to "the first element" and a voltage induced by a signal input to "the first element." "A current of a second element" includes all a current applied to "the second element" and a current induced by a signal input to "the second element." "The first element" and "the second element" are merely exemplary elements, and another element may be described in place of "the first element" and "the second element."

A description such as "a signal input to a third element" includes all a signal applied to an input electrode of the third element and a signal detected from an input electrode of the third element. A signal input to the third element may be affected by the third element. "The third element" is merely an exemplary element, and another element may be described in place of "the third element."

A description such as "a signal output from a fourth element" includes all a signal applied to an output electrode of the fourth element and a signal detected from an output electrode of the fourth element. A signal input to the fourth element may be affected by another element other than the fourth element. "The fourth element" is merely an exemplary element, and another element may be described in place of "the fourth element." A term, such as "overlapping," refers to disposition on a normal direction of a surface of a component without being limited to disposition on or below the surface.

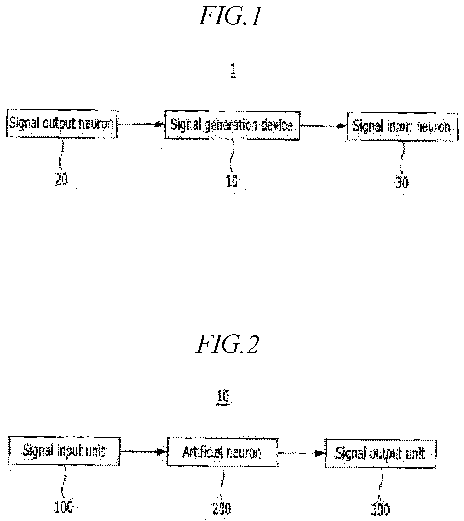

FIG. 1 is an exemplary block diagram of a signal transmission system 1 according to one embodiment of the present disclosure.

Referring to FIG. 1, the signal transmission system 1 may include a signal generation device 10, a signal output neuron 20 and a signal input neuron 30. The signal transmission system 1 may be configured to transmit a spike signal.

The signal generation device 10 may be a device including an artificial neuron. A spike signal may be output from the signal generation device 10. The spike signal output from the signal generation device 10 may be an artificial spike signal. The signal generation device 10 may be configured to detect a spike signal output from the signal output neuron 20. The signal transmission device 10 may be configured to transmit the spike signal to the signal input neuron 30.

The signal output neuron 20 may include a biological neuron. The signal output neuron 20 may include a biological neuron of an animal. The signal output neuron 20 may include a neuron extracted from a brain of a rat. A spike signal may be output from the signal output neuron 20. The spike signal output from the signal output neuron 20 may be a biological spike signal.

The signal input neuron 30 may include a biological neuron. The signal input neuron 30 may include a biological neuron of an animal. The signal input neuron 30 may include a neuron extracted from a brain of a rat. The signal input neuron 30 may be configured to receive an output signal.

FIG. 2 is an exemplary block diagram of a signal generation device 10 according to one embodiment of the present disclosure.

Referring to FIG. 2, the signal generation device 10 may include a signal input unit 100, an artificial neuron 200, and a signal output unit 300.

The signal input unit 100 may be configured to detect a spike signal output from the signal output neuron 20 shown in FIG. 1. A spike signal output from the signal output neuron 20 may be an input spike signal. The input spike signal may be converted by the signal input unit 100 to generate an input signal. The signal input unit 100 may be configured to output an input signal. The input signal output from the signal input unit 100 may be a conversion input signal. The input signal output from the signal input unit 100 may be an integration input signal.

The artificial neuron 200 may be connected to the signal input unit 100. The artificial neuron 200 may be configured to receive the input signal output from the signal input unit 100. A voltage may be applied to the artificial neuron 200, or a signal may be input to induce a voltage. In case that a voltage of the artificial neuron 200 is greater than or equal to a reference voltage, the artificial neuron 200 may output a spike signal. The spike signal output from the artificial neuron 200 may be an artificial spike signal. The spike signal output from the artificial neuron 200 may be an output spike signal.

The signal output unit 300 may be connected to the artificial neuron 200. The signal output unit 300 may be configured to receive the output spike signal output from the artificial neuron 200. The output spike signal may be converted by the signal output unit 300 to generate an output signal. The signal output unit 300 may be configured to transmit the output signal to the signal input neuron 30 shown in FIG. 1. The output signal transmitted to the signal input neuron 30 may be a conversion output signal. The output signal transmitted to the signal input neuron 30 may be an integration output signal.

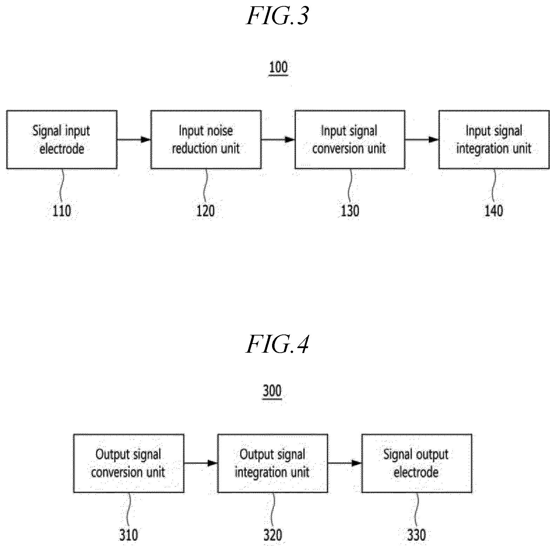

FIG. 3 is an exemplary block diagram of a signal input unit 100 according to one embodiment of the present disclosure.

Referring to FIG. 3, the signal input unit 100 may include a signal input electrode 110, an input noise reduction unit 120, an input signal conversion unit 130, and an input signal integration unit 140.

The signal input electrode 110 may be configured to receive the input spike signal output from the signal output neuron 20 shown in FIG. 1. The signal input electrode 110 may be configured to transmit the input spike signal. The signal input electrode 110 may be a microelectrode array. The signal input electrode 110 may be a two-dimensional microelectrode array or a three-dimensional microelectrode array. The input spike signal detected and transmitted by the signal input electrode 110 may include noise. The input spike signal may be a voltage signal.

The input noise reduction unit 120 may be connected to the signal input electrode 110. The input noise reduction unit 120 may be configured to receive the input spike signal detected by the signal input electrode 110. The input noise reduction unit 120 may be configured to reduce noise of the input spike signal. The input noise reduction unit 120 may be configured to output an input spike signal with reduced noise. In addition, the input noise reduction unit 120 may be configured to amplify the input spike signal. The input noise reduction unit 120 may be configured to output an input spike signal with reduced noise and the signal amplified.

The input signal conversion unit 130 may be connected to the input noise reduction unit 120. The input signal conversion unit 130 may be configured to receive the input spike signal. The input spike signal input to the input signal conversion unit 130 may be an input spike signal with reduced noise. The input signal conversion unit 130 may be configured to convert a received signal. The input signal conversion unit 130 may be configured to output an input signal. The input signal may be different from the input spike signal. The input signal output from the input signal conversion unit 130 may be a conversion input signal.

The input signal integration unit 140 may be connected to the input signal conversion unit 130. The input signal integration unit 140 may be configured to receive an input signal. The input signal may be integrated by the input signal integration unit 140. The input signal integration unit 140 may be configured to transmit the input signal. The input signal transmitted from the input signal integration unit 140 may be an integration input signal.

In one embodiment of the present disclosure, the signal input electrode 110, the input noise reduction unit 120, and the input signal conversion unit 130 may be provided in a plurality. A plurality of conversion input signals output from the plurality of input signal conversion units 130 may be integrated by the input signal integration unit 140.

FIG. 4 is an exemplary block diagram of a signal output unit 300 according to one embodiment of the present disclosure.

Referring to FIG.4, the signal output unit 300 may include an output signal conversion unit 310, an output signal integration unit 320 and a signal output electrode 330.

The output signal conversion unit 310 may be configured to receive an output spike signal. The output signal conversion unit 310 may be configured to convert a received signal. The output signal conversion unit 310 may be configured to output a conversion output signal. The conversion output signal may be different from the output spike signal.

The output signal integration unit 320 may be connected to the output signal conversion unit 310. The output signal integration unit 320 may be configured to receive a conversion output signal. The output signal integration unit 320 may be configured to integrate an output signal. The output signal integration unit 320 may be configured to transmit an output signal. The output signal transmitted from the output signal integration unit 320 may be an integration output signal.

In one embodiment of the present disclosure, the input signal integration unit 140, the artificial neuron 200 and the output signal conversion unit 310 may be provided in a plurality. A plurality of conversion output signals output from the plurality of output signal conversion units 310 may be integrated by the output signal integration unit 320.

The signal output electrode 330 may be connected to the output signal integration unit 320. The signal output electrode 330 may be configured to receive an output signal. The signal output electrode 330 may be configured to transmit the output signal to the signal input neuron 30 shown in FIG. 1. The output signal transmitted from the signal output electrode 330 may be an integration output signal. The signal output electrode 330 may be a microelectrode array. The signal output electrode 330 may be a two-dimensional microelectrode array or a three-dimensional microelectrode array.

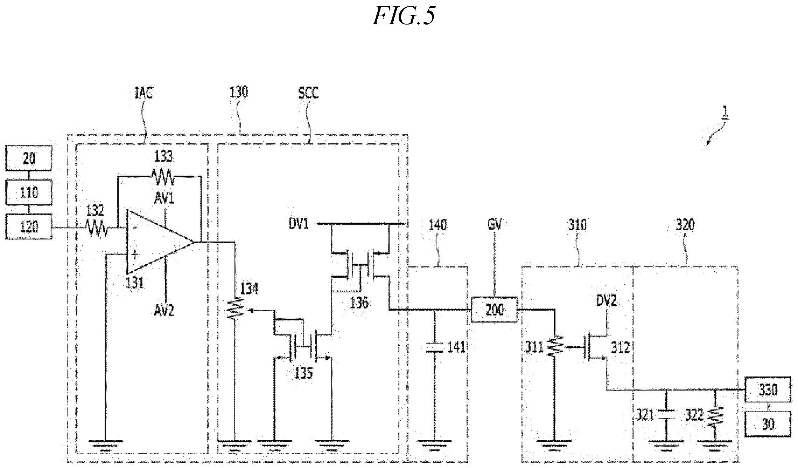

FIG. 5 is an exemplary circuit diagram of a signal processing system 1 according to one embodiment of the present disclosure.

Referring to FIG. 5, the input signal conversion unit 130 may include an input inverting amplifier circuit IAC and an input signal conversion circuit SCC. The input inverting amplifier circuit IAC may include an input operational amplifier 131, an input series resistor 132 and an input parallel resistor 133. The input signal conversion circuit SCC may include an input adjustment resistor 134, a first current mirror 135 and a second current mirror 136. The input signal integration unit 140 may include an input capacitor 141. The output signal conversion unit 310 may include an output adjustment resistor 311 and an output transistor 312. The output signal integration unit 320 may include an output capacitor 321 and an output resistor 322.

The input operational amplifier 131 may be an operational amplifier. The input operational amplifier 131 may be an OP-AMP. The input operational amplifier 131 may be a DC-coupled high-gain voltage amplifier. The input operational amplifier 131 may include an inverting input electrode, a non-inverting input electrode, a signal output electrode, a power input electrode and a power output electrode.

The input series resistor 132, the input parallel resistor 133, and the output resistor 322 may be resistors. The input series resistor 132, the input parallel resistor 133, and the output resistor 322 may be fixed resistors. The input series resistor 132, the input parallel resistor 133, and the output resistor 322 may each include an input electrode and an output electrode. Resistance values between the input electrode and the output electrode of the input series resistor 132, the input parallel resistor 133, and the output resistor 322, respectively, may be constant.

The input adjustment resistor 134 and the output adjustment resistor 311 may be variable resistors. Resistance values of the input adjustment resistor 134 and the output adjustment resistor 311 may be adjusted. The input adjustment resistor 134 and the output adjustment resistor 311 may include an input electrode, an output electrode and a ground electrode. Resistance values between the input electrode and the output electrode of the input adjustment resistor 134 and the output adjustment resistor 311, respectively, may be adjusted. Resistance values between the input electrode and the ground electrode of the input adjustment resistor 134 and the output adjustment resistor 311, respectively, may be fixed.

Although it is illustrated that the input adjustment resistor 134 and the output adjustment resistor 311 are illustrated to be variable resistors in FIG. 5, the input adjustment resistor 134 and the output adjustment resistor 311 may be fixed resistors. The input adjustment resistor 134 and the output adjustment resistor 311 may each include an input electrode and an output electrode. Resistance values between the input electrode and the output electrode of the input adjustment resistor 134 and the output adjustment resistor 311, respectively, may be constant.

The first current mirror 135 and the second current mirror 135 may be current mirrors. The first current mirror 135 and the second current mirror 136 may be configured to copy a current. The first current mirror 135 and the second current mirror 136 may be configured to output a same current as a received current or a current approximate to the received current.

The first current mirror 135 and the second current mirror 136 may include an input electrode, a copy electrode, a first common electrode, and a second common electrode. In case that a current is applied to or induced in the input electrode of the first current mirror 135 and the second current mirror 136, a current of the input electrode may be copied by the copy electrode. In case that a current is applied to or induced in the input electrode of the first current mirror 135 and the second current mirror 136, a current same as or approximate to the current of the input electrode may be generated by the copy electrode. The first common electrode and the second common electrode of the first current mirror 135 and the second current mirror 136 may be connected to a power having a same voltage.

The input capacitor 141 and the output capacitor 321 may be capacitors. The input capacitor 141 and the output capacitor 321 may be variable capacitors. Capacitances of the input capacitor 141 and the output capacitor 321 may be adjusted. The input capacitor 141 and the output capacitor 321 may include an input electrode and an output electrode.

The output transistor 312 may be a transistor. The output transistor 312 may include a gate electrode, a drain electrode, and a source electrode.

The input electrode of the input series resistor 132 may be connected to the input noise reduction unit 120. The output electrode of the input series resistor 132 may be connected to the inverting input electrode of the input operational amplifier 131, and the input electrode of the input parallel resistor 133.

The non-inverting input electrode of the input operational amplifier 131 may be connected to ground. A first amplifier voltage AV1 may be applied to the power input electrode of the input operational amplifier 131. A second amplifier voltage AV2 may be applied to the power output electrode of the input operational amplifier 131. The signal output electrode of the input operational amplifier 131 may be connected to the output electrode of the input parallel resistor 133.

The input electrode of the input adjustment resistor 134 may be connected to the signal output electrode of the input operational amplifier 131. The ground electrode of the input adjustment resistor 134 may be connected to ground. The output electrode of the input adjustment resistor 134 may be connected to the input electrode of the first current mirror 135.

In one or more embodiments, in case that the input adjustment resistor 134 is a fixed resistor, the input electrode of the input adjustment resistor 134 may be connected to the signal output electrode of the input operational amplifier 131. The output electrode of the input adjustment resistor 134 may be connected to the input electrode of the first current mirror 135.

The first common electrode and the second common electrode of the first current mirror 135 may be connected to ground. The copy electrode of the first current mirror 135 may be connected to the input electrode of the second current mirror 136. A first driving voltage DV1 may be applied to the first common electrode and the second common electrode of the second current mirror 136.

The input electrode of the input capacitor 141 may be connected to the copy electrode of the second current mirror 136 and the artificial neuron 200. The output electrode of the input capacitor 141 may be connected to ground.

The artificial neuron 200 may be a field-effect transistor. A gate voltage GV may be applied to the artificial neuron 200.

The input electrode of the output adjustment resistor 311 may be connected to the artificial neuron 200. The ground electrode of the output adjustment resistor 311 may be connected to ground. The output electrode of the output adjustment resistor 311 may be connected to the gate electrode of the output transistor 312. A second driving voltage DV2 may be applied to the drain electrode of the output transistor 312.

In one or more embodiments, in case that the output adjustment resistor 311 is a fixed resistor, the input electrode of the output adjustment resistor 311 may be connected to the artificial neuron 200, and the gate electrode of the output transistor 312. The output electrode of the output adjustment resistor 311 may be connected to ground.

The input electrodes of the output capacitor 321 and the output resistor 322 may be connected to the drain electrode of the output transistor 312, and the signal output electrode 330.

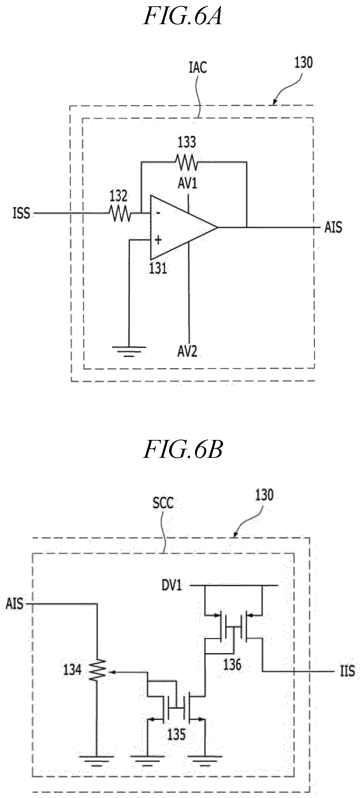

FIG. 6A is an exemplary circuit diagram of an input inverting amplifier circuit IAC according to one embodiment of the present disclosure.

Referring to FIG. 6A, the input inverting amplifier circuit IAC may include an input operational amplifier 131, an input series resistor 132, and the input parallel resistor 133.

The input inverting amplifier circuit IAC may be a circuit configured to amplify a received signal and invert its phase. An input electrode of the input inverting amplifier circuit IAC may be configured to receive an input spike signal ISS. The output electrode of the input inverting amplifier circuit IAC may be configured to output an amplifier input signal AIS. The amplifier input signal AIS may be a signal formed by amplifying the input spike signal ISS and inverting its phase.

The input electrode of the input series resistor 132 may be configured to receive the input spike signal ISS. The input electrode of the input series resistor 132 may be an input electrode of the inverting amplifier circuit IAC. The signal output electrode of the input operational amplifier 131 may be configured to output the amplifier input signal AIS. The signal output electrode of the input operational amplifier 131 may be an output electrode of the inverting amplifier circuit IAC.

The input operational amplifier 131 may be an operational amplifier OP-AMP of the input inverting amplifier circuit IAC. The input series resistor 132 may be an input resistance Rin of the input inverting amplifier circuit IAC. The input parallel resistor 133 may be a feedback resistor Rf of the input inverting amplifier circuit IAC.

FIG. 6B is an exemplary circuit diagram of an input signal conversion circuit SCC according to one embodiment of the present disclosure.

Referring to FIG. 6B, the input signal conversion circuit SCC may include an input adjustment resistor 134, a first current mirror 135, and a second current mirror 136.

The input signal conversion circuit SCC may be a circuit configured to convert a received voltage signal to a current signal. The input electrode of the input signal conversion circuit SCC may be configured to receive the amplified input signal AIS. The amplified input signal AIS may be a voltage signal. The output electrode of the input signal conversion circuit SCC may be configured to output a conversion input signal CIS. The conversion input signal CIS may be a current signal.

The input electrode of the input adjustment resistor 134 may be configured to receive the amplified input signal AIS. The input electrode of the input adjustment resistor 134 may be an input electrode of the input signal conversion circuit SCC. A resistance value of the input adjustment resistor 134 may be adjusted to control a current of the input electrode of the first current mirror 135. The input adjustment resistor 134 may be configured to convert a voltage signal to a current signal.

A current of the input electrode of the first current mirror 135 may be copied by the copy electrode of the first current mirror 135. A current of the copy electrode of the first current mirror 135 may be input to the input electrode of the second current mirror 136 to be copied by the copy electrode of the second current mirror 136. The copy electrode of the second current mirror 136 may be configured to output a conversion input signal CIS. The copy electrode of the second current mirror 136 may be an output electrode of the input signal conversion circuit SCC.

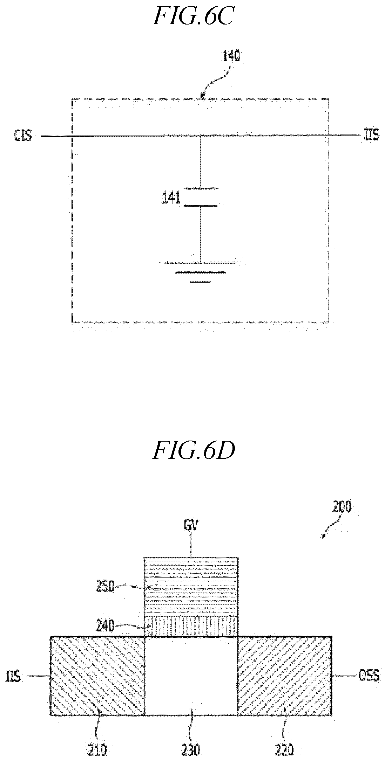

FIG. 6C is an exemplary circuit diagram of an input signal integration unit 140 according to one embodiment of the present disclosure.

Referring to FIG. 6C, the input signal integration unit 140 may include an input capacitor 141.

The input electrode of the input capacitor 141 may be configured to receive a conversion input signal CIS. The input electrode of the input capacitor 141 may be configured to output an integration input signal ISS. In FIG. 6C, the input signal integration unit 140 may be connected to one input signal conversion unit 130.

A voltage value of the integration input signal IIS may dramatically increase in case that a spike occurs in a received conversion input signal CIS. The voltage value of the integration input signal IIS may gradually decrease. A capacitance of the input capacitance 141 may be adjusted to control a rate of decrease in the voltage value of the integration input signal IIS. In one embodiment of the present disclosure, the input capacitance 141 may not be provided.

FIG. 6D is an exemplary illustration of an artificial neuron 200 according to one embodiment of the present disclosure.

Referring to FIG. 6D, the artificial neuron 200 may include an input semiconductor unit 210, an output semiconductor unit 220, a floating semiconductor unit 230, a gate insulating layer 240, and a gate electrode 250. The artificial neuron 200 may be a field-effect transistor.

The input semiconductor unit 210 may be formed by doping a semiconductor. The semiconductor may include a silicon semiconductor, a germanium semiconductor, a Group III-V compound semiconductor or a Group II-VI compound semiconductor. The input semiconductor 210 may be doped to any one type of an n-type and a p-type. The input semiconductor unit 210 may be a drain of the field-effect transistor. The input semiconductor 210 may be connected to the input electrode of the input capacitor 141 shown in FIG. 5. Accordingly, the input semiconductor unit 210 may be configured to receive the integration input signal IIS.

In one embodiment of the present disclosure, the integration input signal IIS may be a signal generated as a current of the conversion input signal CIS increases a voltage of the input semiconductor unit 210. The voltage of the input semiconductor unit 210 may be same as a voltage of the integration input signal IIS. Therefore, in case that a spike occurs in the conversion input signal CIS, a voltage of the input semiconductor unit 210 and a voltage of the integration input signal IIS may dramatically increase.

The output semiconductor unit 220 may be formed by doping the semiconductor. The output semiconductor unit 220 may be spaced apart from the input semiconductor unit 210. The output semiconductor unit 220 may be doped to a same type as the input semiconductor unit 210. The output semiconductor unit 220 may be a source of a field-effect transistor. The output semiconductor unit 220 may be connected to the output adjustment resistor 311 shown in FIG. 5. The output semiconductor unit 220 may be configured to output an output spike signal OSS.

The floating semiconductor unit 230 may be formed by doping the semiconductor. The floating semiconductor unit 230 may be disposed between the input semiconductor unit 210 and the output semiconductor unit 220. The floating semiconductor unit 230 may be doped to a type different from those of the input semiconductor unit 210 and the output semiconductor unit 220. The floating semiconductor unit 230 may be disposed between the input semiconductor unit 210 and the output semiconductor unit 220 to be electrically floating. The floating semiconductor unit 230 may be a channel of a field-effect transistor.

The gate insulating layer 240 may be disposed on the floating semiconductor unit 230. The gate insulating layer 240 may include an insulating material. The gate insulating layer 240 may include, for example, silicon oxide. The gate insulating layer 240 may be a gate oxide layer of a field-effect transistor.

The gate electrode 250 may be disposed on the gate insulating layer 240. The gate electrode 250 may be applied to the gate voltage GV to control the reference voltage. The gate electrode 250 may be a gate of a field-effect transistor. A voltage applied to the gate electrode 250 may be less than or equal to a threshold voltage of the field-effect transistor. In case that the voltage of the input semiconductor unit 210 is less than or equal to the reference voltage, a channel making a contact with the input semiconductor unit 210 and the output semiconductor unit 220 may not be formed on the floating semiconductor unit 230.

In case that a channel is not formed on the floating semiconductor unit 230, the input semiconductor unit 210, the output semiconductor unit 220, and the floating semiconductor unit 230 may function as a capacitance. The input semiconductor unit 210 may function as a first conductor of a capacitance. The output semiconductor unit 220 may function as a second conductor of a capacitance. The floating semiconductor unit 230 may function as an insulating unit of a capacitance.

Depending on capacitances of the input semiconductor unit 210, the output semiconductor unit 220, and the floating semiconductor unit 230, the input signal integration unit 140 may not be provided. That is, the artificial neuron may be configured to integrate a plurality of conversion input signals CIS. For example, the input semiconductor unit 210 may be configured to directly receive a plurality of conversion input signals CIS. In addition, depending on capacitances of the input semiconductor unit 210, the output semiconductor unit 220, and the floating semiconductor unit 230, a rate of decrease in a voltage value of the input semiconductor unit 210 may be controlled.

In one embodiment of the present disclosure, the gate insulating layer 240 and the gate electrode 250 may not be provided.

The artificial neuron 200 may have a latch-down state and a latch-up state. In case that the artificial neuron 200 is in a latch-down state, a current flowing from the input semiconductor unit 210 to the floating semiconductor unit 230 may be limited. In case that the artificial neuron 200 is in a latch-up state, a current flowing from the input semiconductor unit 210 to the floating semiconductor unit 230 may readily flow.

In case that a voltage of the input semiconductor unit 210 is less than the reference voltage, the artificial neuron 200 may be in a latch-down state. In case that a voltage of the input semiconductor unit 210 is greater than or equal to the reference voltage, a state of the artificial neuron 200 may be converted to the latch-up state. The reference voltage may be a drain-source breakdown voltage of the field-effect transistor. The reference voltage may be a latch-up voltage.

In case that the artificial neuron 200 is in a latch-up state, even if a voltage of the input semiconductor unit 210 becomes less than the reference voltage, the latch-up state of the artificial neuron 200 may be maintained. However, in case that a voltage of the input semiconductor unit 210 of the artificial neuron 200 in the latch-up state becomes less than a termination voltage, a state of the artificial neuron 200 may switch to the latch-down state. The termination voltage may be a latch-down voltage.

The input semiconductor unit 210 may be configured to receive an input signal. The input signal may be the integration input signal IIS. A voltage of the input semiconductor unit 210 may be induced by the integration input signal IIS. In case that a voltage of the input semiconductor unit 210 is greater than or equal to a reference voltage, an output spike signal OSS may be generated. The output spike signal OSS may be output from the output semiconductor unit 220.

In case that a voltage of the input semiconductor unit 210 is less than the reference voltage, the artificial neuron 200 may be in a latch-down state. Therefore, an amount of current flowing from the input semiconductor unit 210 to the floating semiconductor unit 230 may be limited. Therefore, a current may be accumulated in the input semiconductor unit 210. Particularly, in case that a current flowing into the input semiconductor unit 210 is greater than a current flowing from the input semiconductor unit 210 to the floating semiconductor unit 230, a current may be accumulated in the input semiconductor unit 210 to increase a voltage of the input semiconductor unit 210. In one embodiment of the present disclosure, the integration input signal IIS may be a signal generated as a current of the conversion input signal CIS is accumulated in the input semiconductor unit 210.

In case that the voltage of the input semiconductor unit 210 is greater than or equal to the reference voltage, a state of the artificial neuron 200 may be switched to the latch-up state. Accordingly, an amount of current flowing from the input semiconductor unit 210 to the floating semiconductor unit 230 may increase. Therefore, a current accumulated in the input semiconductor unit 210 may be discharged. Accordingly, a current flowing into the input semiconductor unit 210 may become less than a current flowing from the input semiconductor unit 210 to the floating semiconductor unit 230. Therefore, a current accumulated in the input semiconductor unit 210 may decrease, and a voltage of the input semiconductor unit 210 may decrease.

In case that a voltage of the input semiconductor unit 210 decreases and becomes less than or equal to a termination voltage, a state of the artificial neuron 200 may switch to the latch-down state. An amount of current flowing from the input semiconductor unit 210 to the floating semiconductor unit 230 may be limited again.

As a result, in case that the voltage of the input semiconductor unit 210 is greater than or equal to the reference voltage, the artificial neuron 200 may switch to the latch-up state, and after lapse of latch-up duration, the artificial neuron 200 may switch to the latch-down state again. The latch-up duration may be short. Therefore, a current accumulated in the input semiconductor unit 210 may flow for a short duration. As a result, an output spike signal OSS may be generated.

The generated output spike signal OSS may be output from the output semiconductor unit 220. The output spike signal OSS may be an artificial spike signal. The output spike signal OSS may be a current signal.

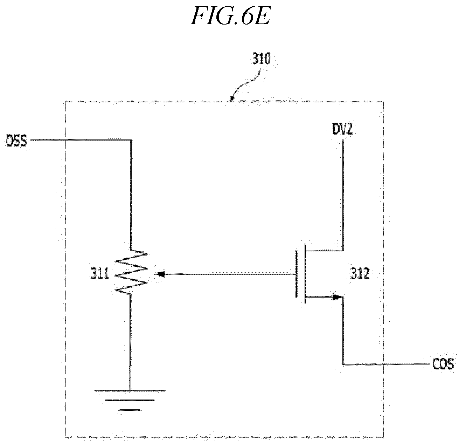

FIG. 6E is an exemplary circuit diagram of an output signal conversion unit 310 according to one embodiment of the present disclosure.

Referring to FIG. 6E, the output signal conversion unit 310 may include an output adjustment resistor 311 and an output transistor 312.

The input electrode of the output adjustment resistor 311 may be configured to receive the output spike signal OSS. A resistance value of the output adjustment resistor 311 may be adjusted to control a voltage applied to the gate electrode of the output transistor 312.

The output spike signal OSS may be a current signal. The output spike signal OSS may be converted to a voltage signal by the output adjustment resistor 311. A signal transmitted to the gate electrode of the output transistor 312 may be a voltage signal.

The drain electrode of the output transistor 312 may be an input electrode. The second driving voltage DV2 may be same as the first driving voltage DV1 shown in FIG. 5.

The source electrode of the output transistor 312 may be configured to output a conversion output signal COS. The source electrode of the output transistor 312 may be an output electrode.

A voltage signal transmitted to the gate electrode of the output transistor 312 may be converted to a current signal flowing from the drain electrode to the source electrode of the output transistor 312. The gate electrode of the output transistor 312 may be a control electrode.

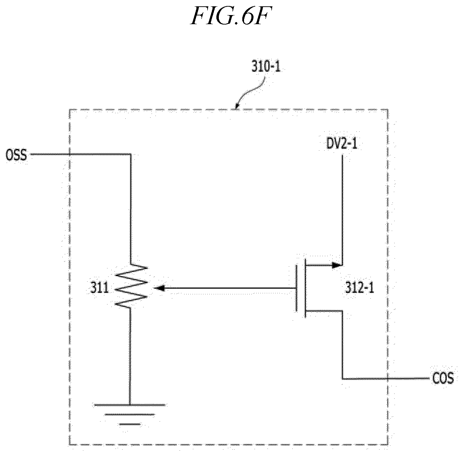

FIG. 6F is an exemplary circuit diagram of an output signal conversion unit 310-1 according to one embodiment of the present disclosure.

Referring to FIG. 6F, the output signal conversion unit 310-1 may include an output adjustment resistor 311 and an output transistor 312-1.

In FIG. 6F, a second driving voltage DV2-1 may be applied to the source electrode of the output transistor 312-1. The source electrode of the output transistor 312-1 may be an input electrode. The second driving voltage DV2-1 may be different from the first driving voltage DV1. The second driving voltage DV2-1 may be less than the first driving voltage DV1. The second driving voltage DV2-1 may be less than 0V, that is a ground voltage.

The drain electrode of the output transistor 312-1 may be configured to output a conversion output signal COS. The drain electrode of the output transistor 312-1 may be an output electrode.

The voltage signal transmitted to the gate electrode of the output transistor 312-1 may be converted to a current signal flowing from the drain electrode to the source electrode of the output transistor 312-1. The gate electrode of the output transistor 312-1 may be a control electrode. The conversion output signal COS may have a negative polarity.

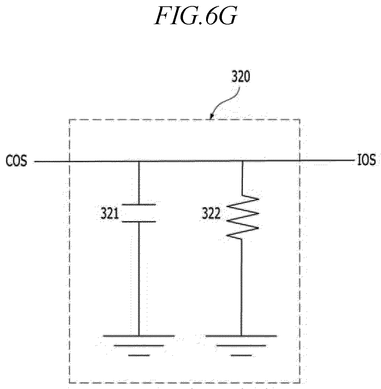

FIG. 6G is an exemplary circuit diagram of an output signal integration unit 320 according to one embodiment of the present disclosure.

Referring to FIG. 6G, the output signal integration unit 320 may include an output capacitor 321 and an output resistor 322.

Input electrodes of the output capacitance 321 and the output resistor 322 may be configured to receive the conversion output signal COS. The input electrodes of the output capacitance 321 and the output resistor 322 may be configured to output an integration output signal IOS. The output signal integration unit 320 may be connected to one output signal conversion unit 310 in FIG. 6G.

The integration output signal IOS may be a spike signal. The integration output signal IOS may vary depending on a capacitance of the output capacitor 321 and a resistance of the output resistor 322. For example, an amplitude and duration of the integration output signal IOS may vary depending on the capacitance of the output capacitor 321 and the resistance of the output resistor 322.

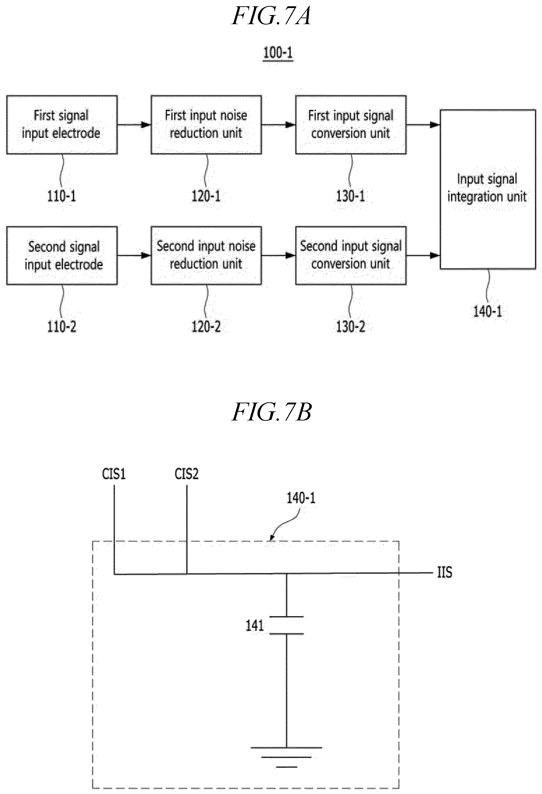

FIG. 7A is an exemplary block diagram of a signal input unit 100-1 according to one embodiment of the present disclosure. FIG. 7B is an exemplary circuit diagram of an input signal integration unit 140-1 according to one embodiment of the present disclosure.

Referring to FIG. 7A, in the signal input unit 100-1, the signal input electrode 110, the input noise reduction unit 120, and the input signal conversion unit 130 shown in FIG. 3 may be provided in a plurality.

A first signal input electrode 110-1 and a second signal input electrode 110-2 may each have a substantially same configuration as the signal input electrode 110 shown in FIG. 3. The first signal input electrode 110-1 and the second signal input electrode 110-2 may be configured to detect an input spike signal output from different signal output neurons. The input spike signal detected by the first signal input electrode 110-1 may be a first input spike signal. The input spike signal detected by the second signal input electrode 110-2 may be a second input spike signal.

Although FIG. 7A illustrates two signal input electrodes 110-1, 110-2, an amount of the plurality of signal input electrodes 110-1, 110-2 is not limited thereto. An amount of the plurality of signal input electrodes provided in one embodiment of the present disclosure 110-1, 110-2 may be 3 or more. The plurality of signal input electrodes 110-1, 110-2 may each be configured to detect a spike signal input from different signal output neurons.

A first input noise reduction unit 120-1 and a second input noise reduction unit 120-2 may each have a substantially same configuration as the input noise reduction unit 120 shown in FIG. 3. The first input noise reduction unit 120-1 may be configured to reduce a noise of the first input spike signal. The second input noise reduction unit 120-2 may be configured to reduce a noise of the second input spike signal.

Although FIG. 7A illustrates two input noise reduction units 120-1, 120-2, an amount of the plurality of input noise reduction units 120-2, 120-2 is not limited thereto. An amount of the plurality of input noise reduction units 120-1, 120-2 provided in one embodiment of the present disclosure may be 3 or more. The plurality of input noise reduction units 120-1, 120-2 may each be configured to reduce a noise of an input spike signal detected by a corresponding signal detection electrode 110-1, 110-2, among the plurality of signal input electrodes 110-1, 110-2.

The first input signal conversion unit 130-1 and the second input signal conversion unit 130-2 may each have a substantially same configuration as the input signal conversion unit 130 shown in FIG. 3. The first input signal conversion unit 130-1 may be configured to receive the first input spike signal with a reduced noise. The second input signal conversion unit 130-2 may be configured to receive the second input spike signal with a reduced noise.

Although FIG. 7A illustrates two input signal conversion units 130-1, 130-2, an amount of the plurality of input signal conversion units 130-1, 130-2 is not limited thereto. An amount of the plurality of input signal conversion units 130-1, 130-2 provided in one embodiment of the present disclosure may be 3 or more. The plurality of input signal conversion units 130-1, 130-2 may each be configured to receive the input spike signal with a reduced noise from a corresponding input noise reduction unit 120-1, 120-2 among the plurality of input noise reduction units 120-1, 120-2.

Referring to FIG. 7A and FIG. 7B, the input signal integration unit 140-1 may be configured to receive a first conversion input signal CIS1 output from the first input signal conversion unit 130-1 and a second conversion input signal CIS2 output from the second input signal conversion unit 130-2. The input signal integration unit 140-1 may be configured to integrate a plurality of conversion input signals CIS1, CIS2 to form an integration input signal IIS. The integration input signal IIS may be transmitted from the input signal integration unit 140-1. The integration input signal may be transmitted to the input semiconductor unit 210 shown in FIG. 6D.

However, an amount of the plurality of conversion input signals CIS1, CIS2 is not limited to 2. An amount of the plurality of conversion input signals CIS1, CIS2 may be 3 or more. As a result, input spike signals output from the plurality of signal output neurons may be integrated in the input signal integration unit 140-1.

In addition, the input signal integration unit 140-1 may not be provided. The plurality of conversion input signals CIS1, CIS2 output from the plurality of input signal conversion units 130-1, 130-2 may be directly transmitted to the input semiconductor unit 210 shown in FIG. 6D. As a result, in one embodiment of the present disclosure, weighted features of a biological neuron may be emulated. The weighted characteristics may refer to a condition in which dendrites of a receiving biological neurons are in contact with axons of a plurality of transmitting biological neurons to allow the receiving biological neurons to receive biological signals from the plurality of transmitting biological neurons. The receiving biological neuron may integrate biological signals received from the plurality of transmitting biological neurons. In case that the integrated biological signal is greater than or equal to a reference value, the biological neuron may be activated to output a spike signal. A biological spike signal of the plurality of biological neurons may be input to the artificial neuron by at least any one of the input signal integration unit 140-1 and the artificial neuron 200.

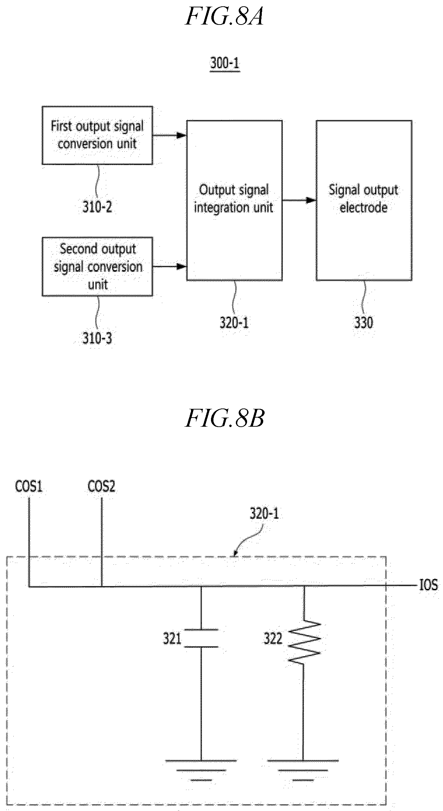

FIG. 8A is an exemplary block diagram of a signal output unit 300-1 according to one embodiment of the present disclosure. FIG. 8B is an exemplary circuit diagram of an output signal integration unit 320-1 according to one embodiment of the present disclosure.

Referring to FIG. 8A, the output signal conversion unit 310 shown in FIG. 4 may be provided in a plurality.

The first output signal conversion unit 310-2 and the second output signal conversion unit 310-3 may each have a same configuration as the output signal conversion unit 310 shown in FIG. 4. The first output signal conversion unit 310-2 may be configured to receive a first output spike signal output from a first artificial neuron. The second output signal conversion unit 310-3 may be configured to receive a second output spike signal output from a second artificial neuron.

Although FIG. 8A illustrates two output signal conversion units 310-2, 310-3, an amount of the plurality of output signal conversion units 310-2, 310-3 is not limited thereto. An amount of the plurality of output signal conversion units 310-2, 310-3 provided in one embodiment of the present disclosure may be 3 or more. The plurality of output signal conversion units 310-2, 310-3 may each be configured to receive an output spike signal output from a corresponding artificial neuron among the plurality of artificial neurons.

Referring to FIG. 8A and FIG. 8B, the output signal integration unit 320-1 may be configured to receive a first conversion output signal COS1 output from the first output signal conversion unit 310-2, and a second conversion output signal COS2 output from the second output signal conversion unit 310-3. The output signal integration unit 320-1 may be configured to integrate the plurality of conversion output signals COS1, COS2 to form an integration output signal IOS. The integration output signal IOS may be transmitted from the output signal integration unit 320-1. The integration output signal may be transmitted to the signal output electrode 330 shown in FIG. 4.

In one or more embodiments, an amount of the plurality of conversion output signals COS1, COS2 is not limited to 2. An amount of the plurality of conversion output signals COS1, COS2 may be 3 or more. As a result, an output spike signal output from the plurality of artificial neurons may be integrated in the output signal integration unit 320.

As a result, in one embodiment of the present disclosure, weighted features of the biological neuron may be emulated. The artificial spike signal of the plurality of artificial neurons may be input to the biological neuron by the output signal integration unit 320.

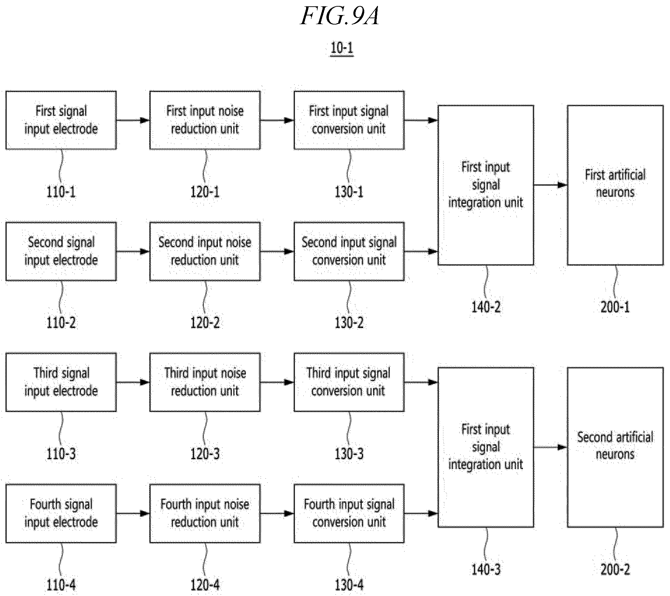

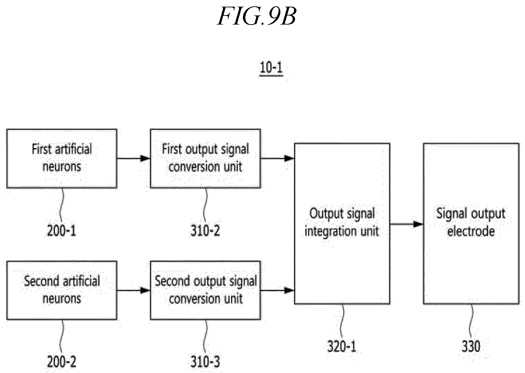

FIG. 9A is an exemplary block diagram of a portion of a signal generation device 10-1 according to one embodiment of the present disclosure. FIG. 9B is an exemplary block diagram of another portion of a signal generation device 10-1 according to one embodiment of the present disclosure.

Referring to FIG. 9A and FIG. 9B, the signal generation device 10-1 may include a plurality of signal input electrodes 110-1, 110-2, 110-3, 1110-4, a plurality of input noise reduction units 120-1, 120-2, 120-3, 120-4, a plurality of input signal conversion units 130-1, 130-2, 130-3, 130-4, a plurality of input signal integration units 140-2, 140-3, a plurality of artificial neurons 200-1, 200-2, and a plurality of output signal conversion units 310-1, 310-2.

The plurality of signal input electrodes 110-1, 110-2, 110-3, 110-4 may each be configured to detect a spike signal received from different signal output neurons.

The plurality of input noise reduction units 120-1, 120-2, 120-3, 120-4 may each be configured to receive an input spike signal detected by a corresponding signal input electrode 110-1, 110-2, 110-3, 110-4 among the plurality of signal input electrodes 110-1, 110-2, 110-3, 110-4.

The plurality of input signal conversion units 130-1, 130-2, 130-3, 130-4 may each be configured to receive an input spike signal with reduced noise from a corresponding input noise reduction unit 120-1, 120-2, 120-3, 120-4 among the plurality of input noise reduction units 120-1, 120-2, 120-3, 120-4.

The first input signal integration unit 140-2 may be configured to receive a first conversion input signal output from the first input signal conversion unit 130-1, and a second conversion input signal output from the second input signal conversion unit 130-2. The first input signal integration unit 140-2 may be configured to transmit a first integration input signal formed by integration of the plurality of conversion input signals. In one or more embodiments, an amount of the plurality of conversion input signals is not limited to 2. An amount of the plurality of conversion input signals may be 3 or more.

The second input signal integration unit 140-3 may be configured to receive a third conversion input signal output from the third input signal conversion unit 130-3, and a fourth conversion input signal output from the fourth input signal conversion unit 130-4. The second input signal integration unit 140-3 may be configured to receive a second integration input signal formed by integration of a plurality of conversion input signals. In one or more embodiments, an amount of the plurality of conversion input signals is not limited to 2. An amount of the plurality of conversion input signals may be 3 or more.

Although FIG. 9A and FIG. 9B illustrate two input signal integration units 140-2, 140-3, an amount of the plurality of input signal integration units 140-2, 140-3 is not limited thereto. An amount of the plurality of input signal integration units 140-2, 140-3 may be 3 or more. In addition, the plurality of input signal integration units 140-2, 140-3 may not be provided.

The first artificial neuron 200-1 and the second artificial neuron 200-2 may be substantially same as the artificial neuron 200 shown in FIG. 5.

The first artificial neuron 200-1 may be configured to receive a first integration input signal transmitted from the first input signal integration unit 140-2. The first artificial neuron 200-1 may be configured to output a first output spike signal.

The second artificial neuron 200-2 may be configured to receive the second integration input signal transmitted from the second input signal integration unit 140-3. The second artificial neuron 200-2 may be configured to output a second output spike signal.

Although FIG. 9A and FIG. 9B illustrate two artificial neurons 200-1, 200-2, an amount of the plurality of artificial neurons 200-1, 200-2 is not limited thereto. An amount of the plurality of artificial neurons 200-1, 200-2 provided in one embodiment of the present disclosure may be 3 or more. The plurality of artificial neurons 200-1, 200-2 may be configured to receive an integration input signal output from a corresponding input signal integration unit 140-2, 140-3 among the plurality of input signal integration units 140-2, 140-3.

As a result, in one embodiment of the present disclosure, weight features of a biological neuron may be emulated. Biological spike signals of the plurality of biological neurons may be input to each artificial neuron by the plurality of input signal integration units 140-2, 140-3. In addition, artificial spike signals of the plurality of artificial neurons may be input to biological neurons by the output signal integration unit 320-1.

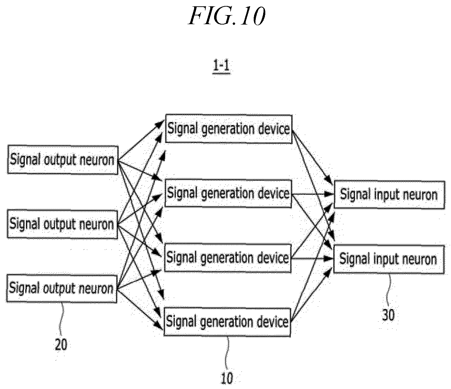

FIG. 10 is an exemplary block diagram of a signal transmission system 1-1 according to one embodiment of the present disclosure.

Referring to FIG. 10, the signal transmission system 1-1 may include a plurality of signal generation devices 10, a plurality of signal output neurons 20, and a plurality of signal input neurons 30.

The plurality of signal generation devices 10 may each be configured to detect an input spike signal output from the plurality of signal output neurons 20. The integration output signal output from each of the plurality of signal generation devices 10 may be transmitted to the plurality of signal input neurons 30.

Therefore, the plurality of signal generation devices 10, the plurality of signal output neurons 20, and the plurality of signal input neurons 30 may consist of a neural network. The signal transmission system 1-1 may be a complex neural network including an artificial neuron and a biological neuron.

Hereinafter, a signal processing method using a signal generation device according to one embodiment of the present disclosure will be described.

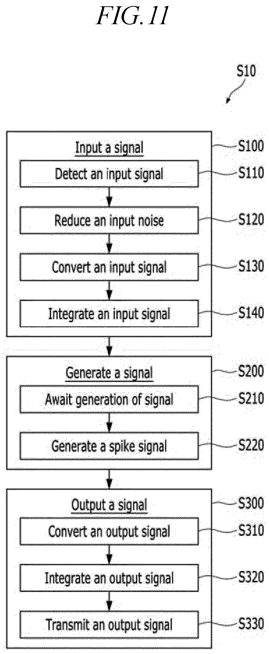

FIG. 11 is an exemplary flow chart of a signal processing method S10 according to one embodiment of the present disclosure.

Referring to FIG. 11, the signal processing method S10 may include inputting a signal S100, generating a signal S200, and outputting a signal S300.

The inputting a signal S100 may include detecting a signal inputS110, reducing an input noise S120, converting a signal input S130, and integrating signal input S140.

In the detecting a signal input S110, an input spike signal output from the signal output neuron 20 may be detected by a signal input electrode 110. The input spike signal may be transmitted to the signal input electrode 110.

In the reducing an input noise S120, an input spike signal may be transmitted to the input noise reduction unit 120. A noise of the input spike signal may be reduced by the input noise reduction unit 120. An input spike signal with a reduced noise may be output from the input noise reduction unit 120. In addition, the input spike signal may be amplified by the input noise reduction unit 120. The input spike signal with a reduced noise and an amplified signal may be output from the input noise reduction unit 120.

In the converting a signal input S130, an input spike signal may be input to the input signal conversion unit 130. A conversion input signal different from the input spike signal may be output from the input signal conversion unit 130.

In the integrating a signal input S140, the conversion input signal may be input to the input signal integration unit 140. An integration input signal may be transmitted to the input signal integration unit 140.

The generating a signal S200 may include awaiting signal generation S210 and generating a spike signal S220.

In the awaiting a signal generation S210, a voltage of the input semiconductor unit 210 is less than a reference voltage. In the awaiting a signal generation S210, the artificial neuron 200 may be in a latch-down state.

In the generating a spike signal S220, a voltage of the input semiconductor unit 210 may be greater than or equal to a reference voltage. In the generating a spike signal S220, an output spike signal may be output from the artificial neuron 200, and a voltage of the input semiconductor unit 210 may be reduced to less than or equal to a reference voltage. A voltage of the input semiconductor unit 210 may be reduced to less than or equal to a termination voltage. In the generating a spike signal S220, the artificial neuron 200 may be in a latch-up state.

Outputting a signal S300 may include converting an output signal S310, integrating an output signa S320, and transmitting an output signal S330.

In the converting an output signal S310, an output spike signal may be input to the output signal conversion unit 310. A conversion output signal different from the output spike signal may be output from the output signal conversion unit 310.

In the integrating an output signal S320, the conversion output signal may be input to the output signal integration unit 320. An integration output signal may be transmitted from the output signal integration unit 320.

In the transmitting an output signal S330, the integration output signal may be transmitted from the signal output electrode 330 to the signal input neuron 30.

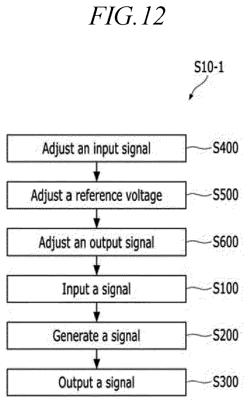

FIG. 12 is an exemplary flow chart of a signal processing method S10-1 according to one embodiment of the present disclosure.

Referring to FIG. 12, the signal processing method S10-1 may include adjusting an input signal S400, adjusting a reference voltage S500, and adjusting an output signal S600.

In the adjusting an input signal S400, at least any one of a resistance value of the input adjustment resistor 134 and a capacitance of the input capacitor 141 may be adjusted to control a conversion input signal. Therefore, operations of the signal generation device 10 and the signal processing method S10-1 may be optimized.

In the adjusting a reference voltage S500, a voltage applied to the gate electrode 250 may be adjusted to control a reference voltage. Therefore, operations of the signal generation device 10 and the signal processing method S10-1 may be optimized.

In the adjusting an output signal S600, at least any one of a resistance value of the output adjustment resistor 311 and a capacitance of the output capacitor 321 may be adjusted to control the conversion output signal. Therefore, operations of the signal generation device 10 and the signal processing method S10-1 may be optimized.

Hereinafter, a signal generation device 10 according to one experimental example of the present disclosure will be described.

### Experimental Example 1

In Experimental Example 1 of the present disclosure, a signal input electrode 110 was disposed on a signal output neuron 20. The signal output neuron 20 may be a neuron extracted from a brain of a rat. The signal input electrode 110 may be a two-dimensional microelectrode array. In addition, an input spike signal output from the signal output neuron 20 was detected by the signal input electrode 110.

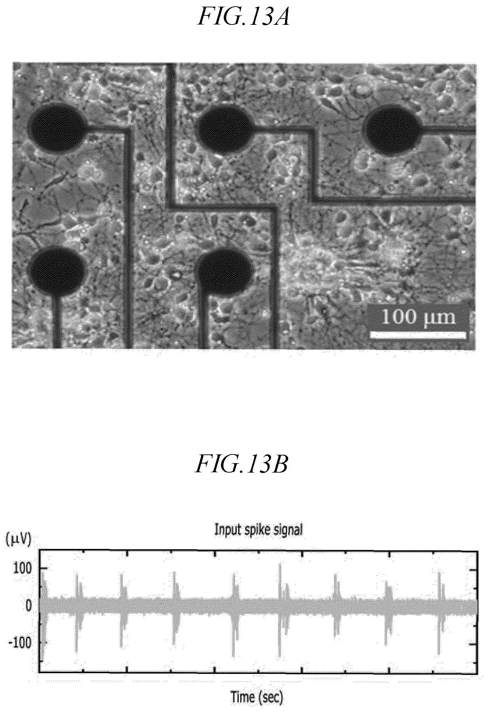

FIG. 13A is a microscopic image of a signal output neuron 20 and a signal input electrode 110 according to one experimental example of the present disclosure.

Referring to FIG. 13A, it can be confirmed in one experimental example of the present disclosure that the signal input electrode 110 is disposed on the signal output neuron 20.

Only images of the signal output neuron 20 and the signal input electrode 110 are shown in FIG. 13A. However, the signal output electrode 330 can also be disposed on the signal input neuron 30 in one experimental example of the present disclosure as shown in FIG. 13A.

FIG. 13B is an input spike signal graph according to one experimental example of the present disclosure.

Referring to FIG. 13B, the signal input electrode 110 according to one embodiment of the present disclosure may be configured to detect an input spike signal output from the signal output neuron 20.

### Experimental Example 2

In Experimental Example 2 of the present disclosure, a graph of an output spike signal OSS output from a neuron 200 depending on a voltage of an input semiconductor unit 210 of the artificial neuron 200 was checked. In Experimental Example 2, a constant current signal with a constant current value, an input signal generated by converting an input spike signal by a signal input unit 100, and an input signal generated by converting a plurality of input spike signals by the signal input unit 100 were input to the input semiconductor unit 210. In Experimental Example 2, an input capacitor 141 was utilized, and a fixed resistor was used as an output adjustment resistor 311.

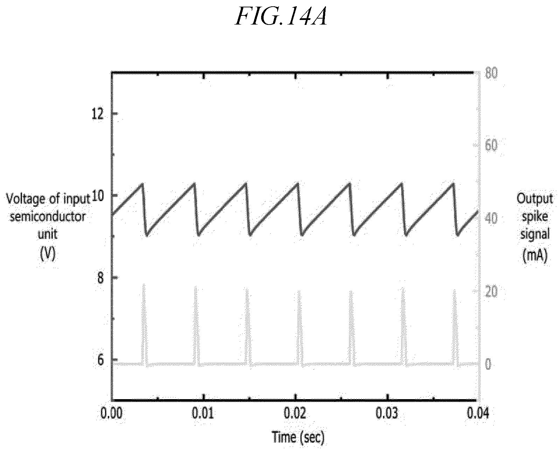

FIG. 14A is a graph of a voltage of an input semiconductor unit 210 and an output spike signal by a constant current signal according to one experimental example of the present disclosure.

Referring to FIG. 14A, in case that a certain amount of current is applied to the input semiconductor unit 210, a voltage of the input semiconductor unit 210 may gradually increase. In case that a voltage of the input semiconductor unit 210 is greater than or equal to a reference voltage, a state of the artificial neuron 200 may switch to a latch-up state, and a current may readily flow from the input semiconductor unit 210 to the floating semiconductor unit 230. The reference voltage may be about 10.3 V.

A current may be discharged from the input semiconductor unit 210 to the floating semiconductor unit 230 to reduce a voltage of the input semiconductor unit 210. In case that a voltage value of the input semiconductor unit 210 becomes less than or equal to a termination voltage, a state of the artificial neuron 200 may switch to a latch-down state to limit a current flowing from the input semiconductor unit 210 to the floating semiconductor unit 230. The termination voltage may be about 9 V.

Therefore, after switching to a latch-up state of the artificial neuron 200, the state may switch to a latch-down state to confirm output of the output spike signal OSS from the artificial neuron 200.

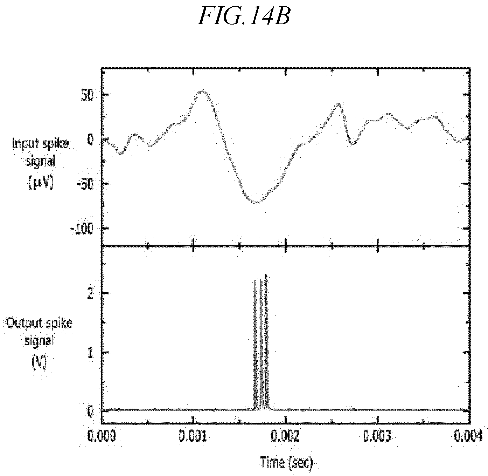

FIG. 14B is a graph of an input spike signal and an output spike signal OSS according to one experimental example of the present disclosure.

Referring to FIG. 14B, an input signal generated by converting the input spike signal by the signal input unit 100 may be input to the artificial neuron 200. In addition, an increase in a voltage of the input semiconductor unit 210 may be induced by an input signal to confirm an output of the output spike signal OSS from the artificial neuron 200. That is, the biological spike signal output from the signal output neuron 20 was input to the artificial neuron 200 through the signal input unit 100, and generation of the input spike signal from the artificial neuron 200 was confirmed.

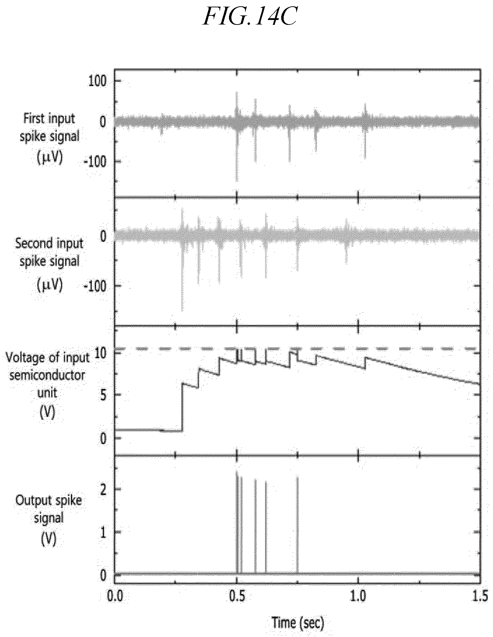

FIG. 14C is a graph of a plurality of input spike signals, and a voltage of an input semiconductor unit 210 and an output spike signal OSS by the plurality of input spike signals according to one experimental example of the present disclosure.

Referring to FIG. 14C, an input signal generated by converting the plurality of input spike signals by the signal input unit 100-1 may be input to the artificial neuron 200. In addition, an induced increase in a voltage of the input semiconductor unit 210 may be confirmed. In case that a voltage value of the input semiconductor unit 210 becomes greater than or equal to a reference voltage indicated by a dotted line, output of an output spike signal OSS from the artificial neuron 200 may be confirmed.

That is, it can be confirmed that a first input spike signal is detected by the first signal input electrode 110-1, and a second input spike signal is detected by the second signal input electrode 110-2. In addition, it can be confirmed that a first conversion input signal CIS1 is output through the first input signal conversion unit 130-1, and a second conversion input signal CIS2 is output through the second input signal conversion unit 130-2. Furthermore, it can be confirmed that the first conversion input signal CIS1 and the second conversion input signal CIS2 are input to the input semiconductor unit 210 through the input signal integration unit 140-1. As a result, it can be confirmed that the weighted features are emulated through at least any one of the input signal integration unit 140-1 and the artificial neuron 200.

### Experimental Example 3

In Experimental Example 3 of the present disclosure, an output spike signal OSS was output from the artificial neuron 200. The output spike signal OSS was converted to an integration output signal by the signal output unit 300 to be transmitted to the signal input neuron 30. In addition, a signal input neuron spike signal defined by a spike signal output from the signal input neuron 30 was confirmed. In Experimental Example 3, the output signal conversion unit 310 shown in FIG. 6E was used.

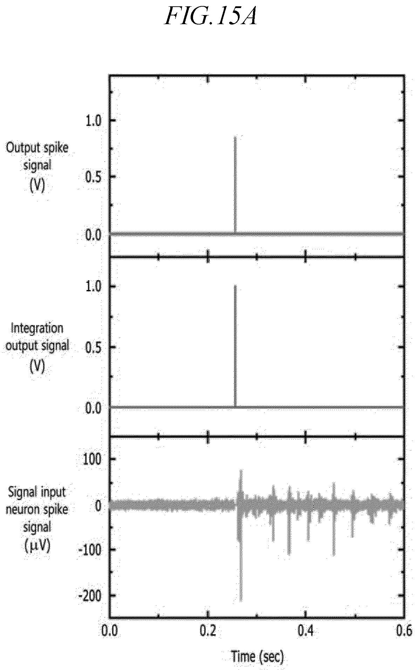

FIG. 15A is a graph of an output spike signal OSS, an integration output signal, and a signal input neuron spike signal according to one experimental example of the present disclosure.

Referring to FIG. 15A, an integration output signal IOS generated by converting the output spike signal by the signal output unit is input to the signal input neuron 30, and it can be confirmed that the signal input neuron spike signal is output from the signal input neuron 30. That is, an artificial spike signal output from the artificial neuron 200 was input to the signal input neuron 30 through the signal output unit 300, and generation of a biological spike signal from the signal input neuron 30 was confirmed.

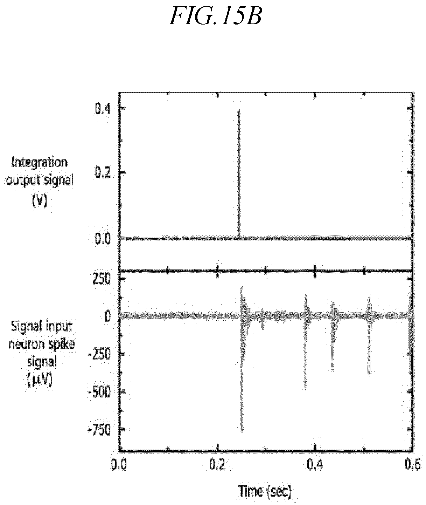

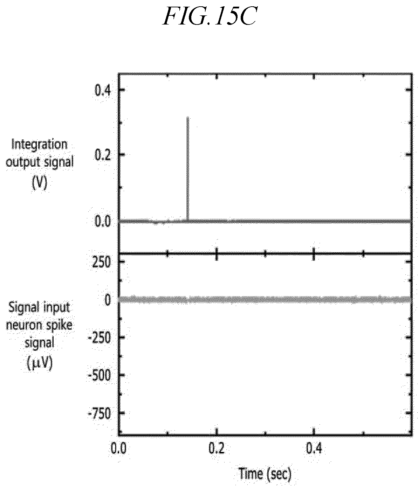

FIG. 15B and FIG. 15C are graphs of an integration output signal and a signal input neuron spike signal according to one experimental example of the present disclosure.

Referring to FIG. 15B and FIG. 15C, it can be confirmed that in case that the integration output signal IOS is greater than or equal to a reference value, a biological spike signal is generated from the signal input neuron 30, and in case that the integration output signal IOS is less than a reference value, no biological spike signal is generated from the signal input neuron 30. Accordingly, in case that the integration output signal IOS is formed by integration of the first conversion output signal COS1 and the second conversion output signal COS2 and the first conversion output signal COS1 and the second conversion output signal COS2 simultaneously output spike signals, a biological spike signal may be generated from the signal input neuron 30.

However, in case that the integration output signal IOS is formed by integration of the first conversion output signal COS1 and the second conversion output signal COS2 and only one of the first conversion output signal COS1 and the second conversion output signal COS2 outputs a spike signal, no biological spike signal may be generated from the signal input neuron 30. As a result, it can be confirmed that the weighted features are emulated through the output signal integration unit 320-1.

### Experimental Example 4

In Experimental Example 4 of the present disclosure, an output spike signal OSS was output from the artificial neuron 200. The output spike signal OSS was converted to an integration output signal by the signal output unit 300 to be transferred to the signal output neuron 30. In addition, a signal input neuron spike signal defined by a spike signal output from the signal input neuron 30 was confirmed. In Experimental Example 4, the output signal conversion unit 310-1 shown in FIG. 6F was utilized.

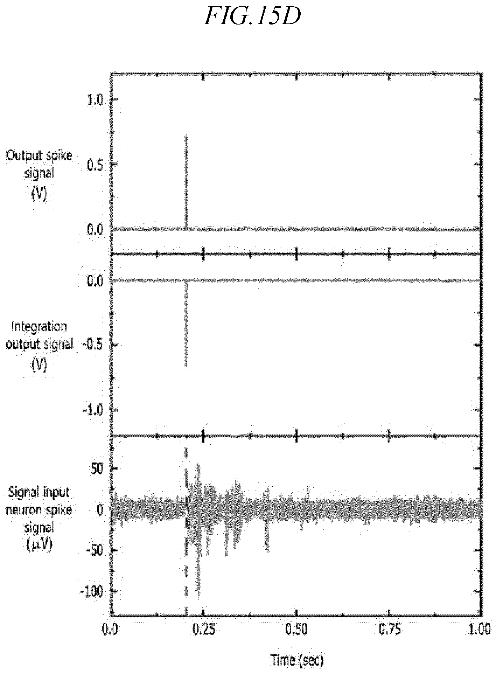

FIG. 15D is a graph of an output spike signal OSS, an integration output signal, and a signal input neuron spike signal according to one experimental example of the present disclosure.

Referring to FIG. 15D, an integration output signal IOS having a negative polarity generated by converting the output spike signal by the signal output unit 300 was input to the signal input neuron 30, and output of a signal input neuron spike signal from the signal input neuron 30 was confirmed. That is, it can be confirmed that the artificial spike signal having a negative polarity output from the artificial neuron 200 was input to the signal input neuron 30 through the signal output unit 300 to generate a biological spike signal from the signal input neuron 30.

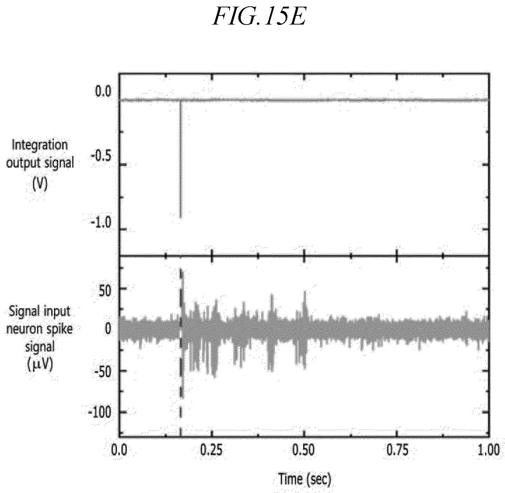

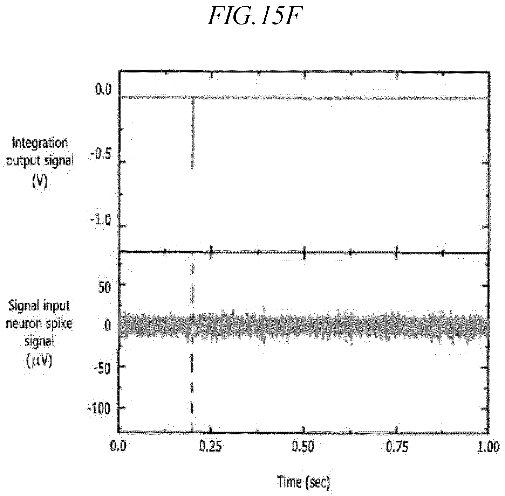

FIG. 15E and FIG. 15F are graphs of an integration output signal and a signal input neuron spike signal according to one experimental example of the present disclosure.

Referring to FIG. 15E and FIG. 15F, it can be confirmed that in case that an absolute value of the integration output signal IOS having a negative polarity is greater than or equal to a reference value, a biological spike signal is generated from the signal input neuron 30, and in case that the absolute value of the integration output signal IOS is less than the reference value, no biological spike signal is generated from the signal input neuron 30. Accordingly, in case that the integration output signal IOS having a negative polarity is formed by integration of a first conversion output signal COS1 having a negative polarity and a second conversion output signal COS2 having a negative polarity and the first conversion output signal COS1 having a negative polarity and the second conversion output signal COS2 having a negative polarity simultaneously output spike signals, a biological spike signal may be generated from the signal intput neuron 30.

### Experimental Example 5

In Experimental Example 5 of the present disclosure, the input spike signal was output from the signal output neuron 20. The input spike signal was converted to an input signal by the signal input unit 100 to be input to the artificial neuron 200. An increase in a voltage of the input semiconductor unit 210 was induced by the input signal to output an output spike signal from the artificial neuron 200. The output spike signal was converted to an integration output signal IOS by the signal output unit 300 to be input to the signal input neuron 30. In addition, the signal input neuron spike signal from the signal input neuron 30 was confirmed.

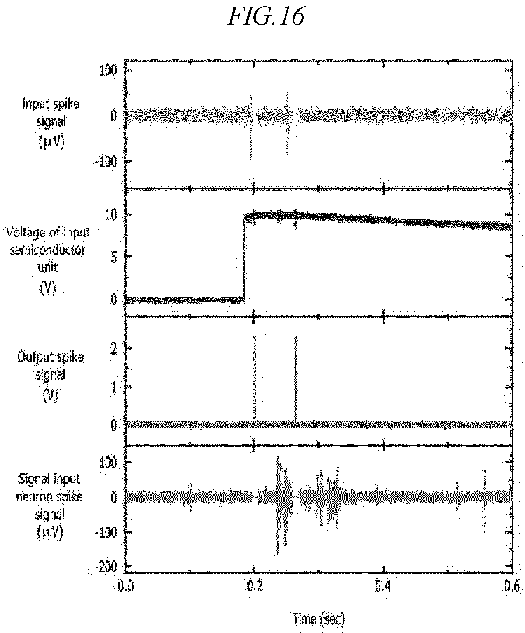

FIG. 16 is a graph of an input spike signal, a voltage of an input semiconductor unit 210, an output spike signal, and a signal input neuron spike signal according to one experimental example of the present disclosure.

Referring to FIG. 16, it can be confirmed that the input spike signal, the output spike signal, and the signal input neuron spike signal are sequentially generated. That is, the input spike signal output from the signal output neuron 20 was input to the signal input neuron 30 through the signal generation device 10.

Hereinafter, effects of the present disclosure will be described with reference to a schematic diagram of a signal generation device according to the present disclosure.

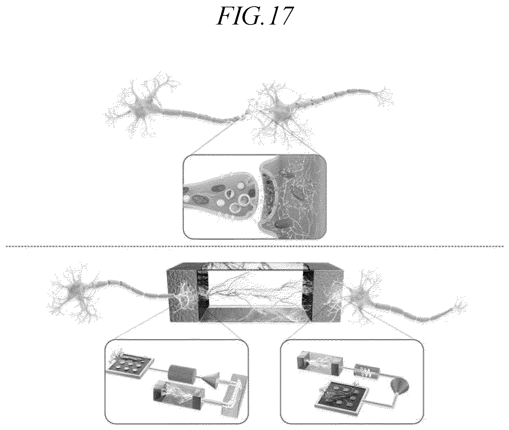

FIG. 17 is an exemplary schematic diagram illustrating a signal transmission effects by a signal generation device 10 of the present disclosure.

Referring to FIG. 17, in the upper part of FIG.17, a chemical signal may be transmitted through neurotransmitters among a plurality of biological neurons. Chemical signaling through the neurotransmitters may depend on a state of a biological neuron. In case that a plurality of biological neurons are in healthy state, the chemical signaling may be properly performed. However, in case that a plurality of biological neurons are in a damaged state, the chemical signaling may be improperly performed.

For example, in case that neurotransmitters, for example a dopamine receptor, of the biological neurons are damaged, a chemical signal may not be transmitted even if the neurotransmitters are transferred. Damage on the dopamine receptors of the biological neurons may lead to neurological disorders, such as movement disorder, Parkinson's disease, depression, and anxiety disorder.

Meanwhile, in the lower part of FIG. 17, an electric signal may be transmitted among a plurality of biological neurons through the signal generation device 10. The electric signal transmission through the signal generation device 10 may not depend on a state of the biological neuron. For example, even in case that for example, the dopamine receptors of the plurality of biological neurons are in a damaged state, the electric signal may be transmitted among the plurality of biological neurons through the signal generation device 10.

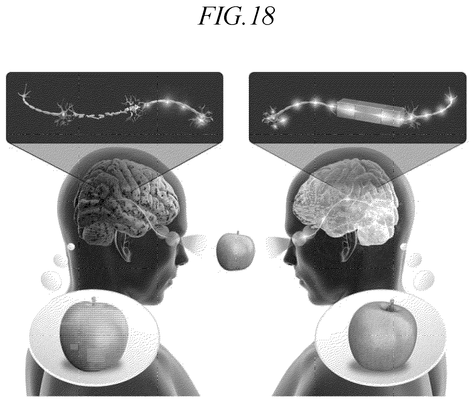

FIG. 18 is an exemplary schematic diagram illustrating a biological neuron replacement function of a signal generation device 10 of the present disclosure.

Referring to FIG. 18, in the left part of FIG. 18, a portion of the plurality of biological neurons may be damaged. Meanwhile, in the right part of FIG. 18, the damaged portion among the plurality of biological neurons may be replaced by the signal generation device 10 of the present disclosure.

In the left part of FIG. 18, a portion of the plurality of biological neurons may be damaged, and a plurality of biological neurons may fail to properly process visual information. However, in the right part of FIG. 18, a signal generation device 10 of the present disclosure may replace the damaged portion among the plurality of biological neurons to function as artificial neurons. Accordingly, in the right part of FIG. 18, the plurality of biological neurons and the signal generation device 10 may properly process visual information.

However, although a single signal generation device 10 is illustrated in the right part of FIG. 18, this may merely be an exemplary configuration. In one embodiment of the present disclosure, the plurality of signal generation devices 10 may be disposed to replace the plurality of damaged biological neurons.

While certain embodiments of the present disclosure have been described above, anyone ordinarily skilled in the art to which the present disclosure pertains shall appreciate that there may be a variety of modifications and permutations of the present disclosure without departing from the technical ideas and scopes of the present disclosure that are defined in the appended claims. Moreover, it shall be appreciated that the disclosed embodiments are not intended to restrict the present disclosure thereto and that every technical idea within the appended claims and their equivalents is interpreted to be included in the scope of the present disclosure.

## Claims

1. A spike signal generation device comprising:
a signal input unit comprising a signal input electrode configured to transmit an input spike signal output from a signal output neuron, and an input signal conversion unit configured to receive the input spike signal to output an input signal different from the input spike signal, the input signal conversion unit comprising an input operational amplifier and an input adjustment resistor;
an artificial neuron comprising an input semiconductor unit configured to receive the input signal, an output semiconductor unit spaced apart from the input semiconductor unit, and a floating semiconductor unit disposed between the input semiconductor unit and the output semiconductor unit, wherein in case that a voltage of the input semiconductor unit is equal to or greater than a reference voltage, the output semiconductor unit outputs an output spike signal; and
a signal output unit comprising an output signal conversion unit configured to receive the output spike signal to output an output signal different from the output spike signal, and a signal output electrode configured to transmit the output signal to a signal input neuron, the output signal conversion unit comprising an output adjustment resistor.

2. The spike signal generation device of claim 1,
wherein the input signal conversion unit further comprises an input series resistor and an input parallel resistor,
wherein an input electrode of the input series resistor is configured to receive the input spike signal,
wherein an output electrode of the input series resistor is connected to an inverting input electrode of the input operational amplifier,
wherein an input electrode of the input parallel resistor is connected to the inverting input electrode of the input operational amplifier,
wherein an output electrode of the input parallel resistor is connected to a signal output electrode of the input operational amplifier, and
wherein a non-inverting input electrode of the input operational amplifier is connected to ground.

3. The spike signal generation device of claim 2,
wherein the output signal conversion unit further comprises an output transistor, and
wherein a control electrode of the output transistor is connected to an output electrode of the output adjustment resistor.

4. The spike signal generation device of claim 3,
wherein the signal input unit further comprises an input signal integration unit configured to transmit the input signal to the input semiconductor unit, the input signal integration unit comprising an input capacitor, wherein an input electrode of the input capacitor is connected to the input semiconductor unit, and
wherein the signal output unit further comprises an output signal integration unit configured to transmit the output signal to the signal output electrode, the output integration unit comprising an output capacitor and an output resistor, wherein an input electrode of the output capacitor and an input electrode of the output resistor are connected to an output electrode of the output transistor.

5. The spike signal generation device of claim 4,
wherein the input adjustment resistor and the output adjustment resistor are variable resistors whose resistance values can be adjusted,
wherein the signal output electrode of the input operational amplifier is connected to an input electrode of the input adjustment resistor, and
wherein the output semiconductor unit is connected to an input electrode of the output adjustment resistor.

6. The spike signal generation device of claim 5,
wherein the floating semiconductor unit is doped into any one type of an n-type and a p-type, and
wherein the input semiconductor unit and the output semiconductor unit are doped into one of the n-type and the p-type, different from that of the floating semiconductor unit.

7. The spike signal generation device of claim 5,
wherein the artificial neuron further comprises a gate insulating layer disposed on the floating semiconductor unit, and a gate electrode disposed on the gate insulating layer, wherein a voltage is applied to the gate electrode to adjust the reference voltage.

8. The spike signal generation device of claim 7,
wherein the input signal conversion unit further comprises a first current mirror and a second current mirror configured to copy a received current,
wherein an input electrode of the first current mirror is connected to an output electrode of the input adjustment resistor,
wherein an input electrode of the second current mirror is connected to a copy electrode of the first current mirror,
wherein a copy electrode of the second current mirror is connected to the input semiconductor unit,
wherein the copy electrode of the first current mirror is configured to copy a current of the input electrode of the first current mirror, and
wherein the copy electrode of the second current mirror is configured to copy a current of the input electrode of the second current mirror.

9. The spike signal generation device of claim 8,
wherein an output electrode of the input capacitor, an output electrode of the output capacitor, and an output electrode of the output resistor are connected to ground.

10. The spike signal generation device of claim 8,
wherein the signal input electrode and the signal output electrode are microelectrode arrays.

11. The spike signal generation device of claim 8,
wherein input signal conversion unit is provided in a plurality,
wherein the input signal output from a first input signal conversion unit among the plurality of input signal conversion units is a first conversion input signal,
wherein the input signal output from a second input signal conversion unit among the plurality of input signal conversion units is a second conversion input signal, and
wherein the input signal transmitted from the output signal integration unit to the input semiconductor unit is an integration input signal formed by integration of the first conversion input signal and the second conversion input signal.

12. The spike signal generation device of claim 8,
wherein the output signal conversion unit is provided in a plurality,
wherein the output signal output from a first output signal conversion unit among the plurality of output signal conversion units is a first conversion output signal,
wherein the output signal output from a second output signal conversion unit among the plurality of output signal conversion units is a second conversion output signal, and
wherein the output signal transmitted from the output signal integration unit to the signal output electrode is an integration output signal formed by integration of the first conversion output signal and the second conversion output signal.

13. A spike signal processing method comprising:
inputting a signal, wherein in the inputting a signal, an input spike signal output from a signal output neuron is detected by a signal input electrode, the input spike signal is input to an input signal conversion unit comprising an input operational amplifier and an input adjustment resistor, the input signal conversion unit outputs a conversion input signal different from the input spike signal, the conversion input signal is input to an input signal integration unit comprising an input capacitor, and the input signal integration unit transmits an integration input signal;
generating a signal, wherein in the generating a signal, the integration input signal is input to an artificial neuron comprising an input semiconductor unit, an output semiconductor unit spaced apart from the input semiconductor unit, and a floating semiconductor unit disposed between the input semiconductor unit and the output semiconductor unit, and in case that a voltage of the input semiconductor unit is greater than or equal to a reference voltage, and an output spike signal is output from the artificial neuron; and
outputting a signal, wherein in the outputting a signal, the output spike signal is input to an output signal conversion unit comprising an output adjustment resistor, a conversion output signal different from the output spike signal is output from the output signal conversion unit, the conversion output signal is input to an output signal integration unit comprising an output capacitor and an output resistor, an integration output signal is transmitted from the output signal integration unit, and the integration output signal is transmitted from a signal output electrode to a signal input neuron.

14. The spike signal processing method of claim 13,
wherein the generating a signal comprises:
awaiting signal generation, wherein in the awaiting signal generation, a voltage of the input semiconductor unit is less than the reference voltage, and
generating a spike signal, wherein in the generating a spike signal, the voltage of the input semiconductor unit becomes greater than or equal to the reference voltage to output an output spike signal.

15. The spike signal processing method of claim 14,
wherein the artificial neuron further comprises a gate insulating layer disposed on the floating semiconductor unit, and a gate electrode disposed on the gate insulating layer, and
wherein the method further comprises adjusting a reference voltage, where a voltage is applied to the gate electrode to adjust the reference voltage.
